# EUROPEAN PATENT APPLICATION

(11) **EP 4 439 577 A1**
(43) Date of publication of application: **02.10.2024**
(21) Application number: 24166578.5
(22) Date of filing: 26.03.2024
(51) Int. Cl.: G16H 20/70, G16H 40/63

(54) **SYSTEMS AND METHODS FOR SAMPLING A PRESCRIPTION DIGITAL THERAPEUTIC**

(30) Priority: 27.03.2023 US 202363492462 P
(71) Applicant: Pear Therapeutics (US), Inc., Boston, MA 02109 (US)
(72) Inventor: Maricich, Yuri, Boston (US); Raza, Usman, Boston (US); Gillis, Heather, Boston (US); Datoo, Mohamed, Boston (US); Otis, Craig, Boston (US); Prunler, Brian C., Wayland (US)
(74) Representative: Murgitroyd & Company

(57) **Abstract**

Presented herein are systems and methods of controlling access to user interfaces. A computing system can identify a plurality of user interfaces to present digital therapeutic content to a user to address a condition using one or more of the plurality of user interfaces. The computing system can provide, to a user device associated with the user over a time window, access to a subset of the plurality of user interfaces to present a corresponding portion of the digital therapeutic content to the user. The computing system can determine that interactions by the user satisfy a criterion based on data from the user device. The computing system can provide, to the user device, access to the plurality of user interfaces to present an entirety of the digital therapeutic content to the user, responsive to determining that the interactions satisfy the criterion.

## Description

### CROSS REFERENCES TO RELATED APPLICATIONS

The present application claims priority to U.S. Provisional Patent Application No. 63/492,462, titled "Systems and Methods for Sampling a Prescription Digital Therapeutic," filed March 27, 2023, which is incorporated herein by reference in its entirety.

### FIELD

The present disclosure relates to digital therapeutics and, more particularly, to systems and methods controlling access to user interfaces for digital therapeutics to treat symptoms associated with one or more medical conditions.

### BACKGROUND

Drug therapy may be provided to a subject to alleviate or treat various medical conditions. While drug therapy in combination with cognitive behavioral therapy has proven to be an effective mechanism for treating certain medical conditions, it is not without deficiencies. For example, prescriptions for treatment of conditions may require onboarding of a patient into systems, which may significantly delay treatment. This problem may be particularly pronounced where the patient is suffering from life-threatening diseases or disorders, such as opioid use disorder, where the risk associated with any delay in treatment may prove fatal.

Digital therapeutics may be provided via an application on a computing device in furtherance of addressing the medical conditions of the user. One issue with the provision of digital therapeutics may be with respect to the overall functionality in that the content and interactivity of the application may be limited and restricted to the originally pre-configured capabilities. For example, the application may be configured with a static set of user interfaces to deliver the fixed digital therapeutic content to the user, without any consideration as to the efficacy or engagement with respect to the specific user. This may result in lower or reduced efficacy of the digital therapeutic in addressing the conditions of the user as well as wasted consumption of computing resources and network bandwidth from providing ineffective content. Another issue may be with respect to security and risk in that certain user interfaces and information should be restricted from presentation to the user, without additional checks.

### SUMMARY

To address these and other technical challenges, user access to user interfaces for receiving digital therapeutic content may be regulated and controlled by a computing system. By regulating and controlling user access, a prescription for a sample of the digital therapeutic may be provided to the user. One benefit from sampling in this manner may be to provide the user with access to an earlier time point to at least a portion of the digital therapeutic to resolve or reduce any delays from providing the full digital therapeutic. The delay may, for example, be incurred from the enrollment and on-boarding process for the user or processing by a payor for the prescription of the full digital therapeutic.

In providing a portion of the digital therapeutic as a sample, another advantage may be that the user is able to be placed on the therapy regimen for a shortened time period on a trial basis and may be monitored as to how the user interacts with the digital therapeutic content. During the trial period, the computing system may determine whether the user is engaged with the sample digital therapeutic. Based on the engagement with the sample, the full digital therapeutic content may be modified or personalized for the particular user. The trial period may also allow the user to acclimate to the application as well as the sample digital therapeutic content, prior to being administered with the full digital therapeutic.

Another advantage may be to decrease or mitigate risk for various entities, such as the health care provider prescribing the digital therapeutic, the administrator of the digital therapeutic, and the end user receiving the digital therapeutic content through their computing device. By limiting the user's initial exposure to the sample digital therapeutic, the provider and administrator may be able to identify whether the user is engaged, before providing the user with potentially more difficult tasks as directed via the digital therapeutic. When the user is identified to be engaged with the sample, the digital therapeutic content may be augmented to be more personalized and targeted for the given user.

To regulate and control user access, the application may initially provide the user access to a subset of user interfaces to provide a corresponding portion (or a sampling) of the digital therapeutic. While the user is provided access to the subset of user interfaces, the server may aggregate and collect data about the user indicating engagement or efficacy of the digital therapeutic. The data may also indicate (e.g., by a remote computing system) whether the user is to be provided access to the overall set of user interfaces for the digital therapeutic. Based on the data, the computing system may determine whether to dynamically set the user interfaces available for access by the user to provide additional digital therapeutic. For example, when the data indicates that user is engaged or is otherwise to be provided with the overall set of user interfaces, the computing system may provide the user access to the overall set of the digital therapeutics (or full digital therapeutic). With additional data about the user, the computing system may continue to add functionalities (e.g., in the form of content and interactivity) accessible to the user.

By dynamically controlling the access to user interfaces, the computing system can regulate the consumption of computing resources and network bandwidth, reducing such consumption that otherwise would have been wasted from providing the full set of user interfaces from the beginning. Furthermore, the restricting or limiting access to certain user interfaces may allow the user to be provided with a sample of the digital therapeutic, thereby improving the quality of human-computer interaction (HCI) between the user and the application. This may be used to monitor the user interactions with the digital therapeutic treatment for a shortened time period and then determine whether the user is to be provided access to additional digital therapeutic content. For instance, if the specific user is determined to have engaged with the content, the computing device may provide access to the additional sets of user interfaces for the full digital therapeutic. Conversely, if a given user is determined to have reduced engagement or it is indicated that the user is to receive the full digital therapeutic, the computing system may identify user interfaces that that may be more engaging and targeted for this specific user. In addition, the computing system may reduce the security and related risk for the provider of the digital therapeutic and the user receiving the digital therapeutic.

Aspects of the present disclosure are systems and methods of controlling access to user interfaces. One or more processors can identify a plurality of user interfaces to present digital therapeutic content to a user to address a condition using one or more of the plurality of user interfaces. The one or more processors can provide, to a user device associated with the user over a time window, access to a subset of the plurality of user interfaces to present a corresponding portion of the digital therapeutic content to the user. The one or more processors can receive data from the user device, the data identifying interactions by the user with one or more of the subset of the plurality of user interfaces. The one or more processors can determine that the interactions by the user satisfy a criterion with the corresponding portion of the digital therapeutic content within the time window, based on the data from the user device. The one or more processors can provide, to the user device, access to the plurality of user interfaces to present an entirety of the digital therapeutic content to the user, responsive to determining that the interactions satisfy the criterion.

In some embodiments, the one or more processors can determine that the corresponding portion of the digital therapeutic content is effective at addressing the condition associated with the user within the time period, based on the data. In some embodiments, the one or more processors can determine that second interactions by the user do not satisfy the criterion with the corresponding portion of the digital therapeutic content within the time window, based on second data from the user device. In some embodiments, the one or more processors can refrain, from providing the user device access to the plurality of user interfaces to present the entirety of the digital therapeutic content, responsive to determining that the second interactions do not satisfy the criterion.

In some embodiments, the one or more processors can generate an access code to provide the user device access to the plurality of user interfaces for the entirety of the digital therapeutic content, responsive to determining that the interactions satisfy the criterion. In some embodiments, the one or more processors can provide the user device access to the plurality of user interfaces, responsive to entry of the access code via the user device. In some embodiments, the one or more processors can provide the user device access to a second user interface different from any of the plurality of user interfaces.

In some embodiments, the user may be on a medication to address the condition, in at least partial concurrence with at least one of (i) provision of the subset of the plurality of user interfaces for the portion of the digital therapeutic content or (ii) provision of the plurality of user interfaces for the entirety of the digital therapeutic content. In some embodiments, the condition can include at least one of: substance use disorder, opioid use disorder, chronic insomnia, alcohol use disorder, schizophrenia, generalized anxiety disorder, major depressive disorder, bipolar, posttraumatic stress disorder, acute and chronic pain, migraine, multiple sclerosis, epilepsy, irritable bowel syndrome, specialty gastroenterology, cancer, or cardiovascular disease.

Other aspects of the present disclosure are systems and methods of controlling access to user interfaces. One or more processors can identify a plurality of user interfaces to present digital therapeutic content to a user to address a condition using one or more of the plurality of user interfaces. The one or more processors can provide, to a user device associated with the user, access to a subset of the plurality of user interfaces to present a corresponding portion of the digital therapeutic content to the user. The one or more processors can receive an indication to provide access to the entirety of the digital therapeutic content to the user. The one or more processors can provide, to the user device, access to the plurality of user interfaces to present an entirety of the digital therapeutic content to the user, responsive to receiving the indication to approve access.

In some embodiments, the one or more processors can determine that a time window for providing the user device access to the subset of the plurality of user interfaces has expired. In some embodiments, the one or more processors can restrict the user device from accessing the subset of the plurality of user interfaces, responsive to determining that the time window has expired. In some embodiments, the one or more processors can generate an access code to provide the user device access to the subset of the plurality of user interfaces, responsive to the indication to provide the digital therapeutic content to the user. In some embodiments, the one or more processors can provide the user device access to the subset of the plurality of user interfaces, responsive to entry of the access code via the user device.

In some embodiments, the one or more processors can select, from a second plurality of user interfaces, a second user interface to add to the subset of the plurality of user interfaces based on data from the user device, the data identifying interactions by the user with one or more of the subset of the plurality of user interfaces. In some embodiments, the one or more processors can provide to the user device, access to the second user interface in addition to the subset of the plurality of user interfaces. In some embodiments, the one or more processors can generate an access code to provide the user device access to the plurality of user interfaces for the entirety of the digital therapeutic content. In some embodiments, the one or more processors can provide the user device access to the plurality of user interfaces, responsive to entry of the access code via the user device. In some embodiments, the one or more processors can receive the indication responsive to an approval by a remote service of the provision of the entirety of the digital therapeutic content to the user.

In some embodiments, the user may be on a medication to address the condition, in at least partial concurrence with at least one of (i) provision of the subset of the plurality of user interfaces for the portion of the digital therapeutic content or (ii) provision of the plurality of user interfaces for the entirety of the digital therapeutic content. In some embodiments, the condition can include at least one of: substance use disorder, opioid use disorder, chronic insomnia, alcohol use disorder, schizophrenia, generalized anxiety disorder, major depressive disorder, bipolar, posttraumatic stress disorder, acute and chronic pain, migraine, multiple sclerosis, epilepsy, irritable bowel syndrome, specialty gastroenterology, cancer, or cardiovascular disease.

### DESCRIPTION OF DRAWINGS

FIG. 1 is a schematic view of a system for treating symptoms associated with indications including a prescription digital therapeutic in accordance with an exemplary embodiment of the present disclosure;
FIG. 2 is a schematic view of example components of the prescription digital therapeutic of FIG. 1;
FIG. 3 is a schematic view of example components of the prescription digital therapeutic of FIG. 1;
FIGS. 4A-4D are exemplary GUIs of a prescription digital therapeutic of FIG. 1 displayed on a display of a patient electronic device;
FIG. 5 is a flowchart illustrating a method for implementing a sample prescription digital therapeutic, in accordance with an exemplary embodiment of the present disclosure;
FIG. 6 depicts a flow diagram of a process for controlling access to user interfaces, in accordance with an illustrative embodiment; and
FIG. 7 is a schematic view of an example computing device that may be used to implement the systems and methods described herein.

Like reference symbols in the various drawings indicate like elements

### DETAILED DESCRIPTION

Example embodiments are provided so that this disclosure will be thorough, and will fully convey the scope to those who are skilled in the art. Numerous specific details are set forth such as examples of specific compositions, components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

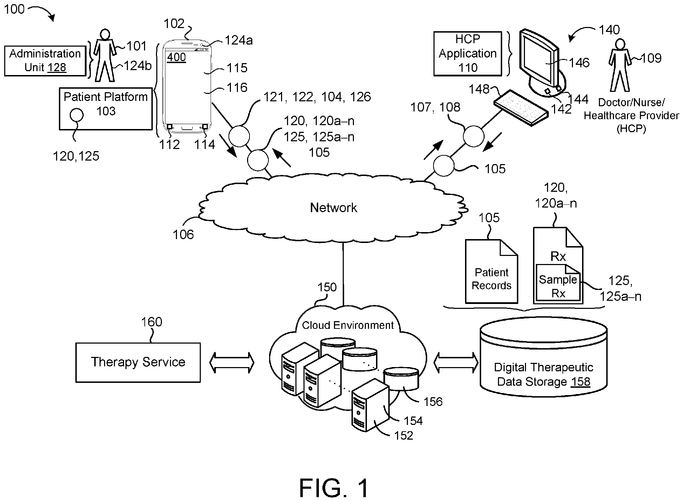

Referring to FIG. 1, in some implementations, a therapy prescription system 100 provides a patient 101 access to one or more prescription digital therapeutics 120, 120a-n (also referred to as full prescription digital therapeutics 120 and digital therapeutics 120) prescribed to the patient 101 and monitors events associated with the patient's 101 interaction with the prescription digital therapeutic 120. The prescription digital therapeutics 120 may include both indication-agnostic digital therapies 120 capable of treating or administered to treat multiple indications, and indication-specific digital therapies 120 administered to treat a specific indication. Although the digital therapeutics 120 are described herein as being "prescription" digital therapeutics, it is understood that, according to some implementations, the digital therapeutics 120 may not require a prescription from a clinician. Rather, in such implementations, the digital therapeutics 120 may be available to a patient without a prescription, and such a non-prescription digital therapeutic 120 nonetheless otherwise functions in accordance with the description of the prescription digital therapeutics 120 described herein. The person using or being administered the digital therapeutics 120 may be referred to as a "user." A "user" may include a patient 101 or any other person using or being administered the digital therapeutics 120, irrespective of whether the digital therapeutics 120 were prescribed to that person.

As used herein, a digital therapy may also be referred to as a digital-therapeutic configured to deliver evidence-based psychosocial intervention techniques for treating a patient with a particular indication (e.g., disease or disorder), as well as symptoms and/or behaviors associated with the particular disease or disorder. Examples of a particular disease, disorder, or indication include substance use disorder, opioid use disorder, chronic insomnia, alcohol use disorder, schizophrenia, generalized anxiety disorder, major depressive disorder, bipolar, posttraumatic stress disorder, acute and chronic pain, migraine, multiple sclerosis, epilepsy, irritable bowel syndrome, specialty gastroenterology, cancer and/or cardiovascular disease or disorder. As one example, the patient 101 may experience one or more disorders and the prescription digital therapeutics 120 may be tailored for addressing one or more symptoms associated with each of the one or more disorders that the patient 101 may experience. As will be discussed in greater detail below, each of the prescription digital therapeutics 120 may include a respective sample prescription digital therapeutic 125 to administer therapeutic content to treat one or more different indications while a prescription for the full prescription digital therapeutic 120 is processed.

In some implementations, the digital therapeutics 120 may include or be combined with traditional drug therapy, which similarly may or may not require a prescription. An authorized healthcare provider (HCP) 109 (e.g., a doctor, nurse, etc.) may prescribe the patient 101 one or more of the prescription digital therapeutics 120 designed to treat symptoms in the patient 101. The HCP 109 may include a physician, nurse, clinician, or other qualified health professionals. The HCP 109 may provide any suitable level of supervision to the patient 101, including little to no supervision.

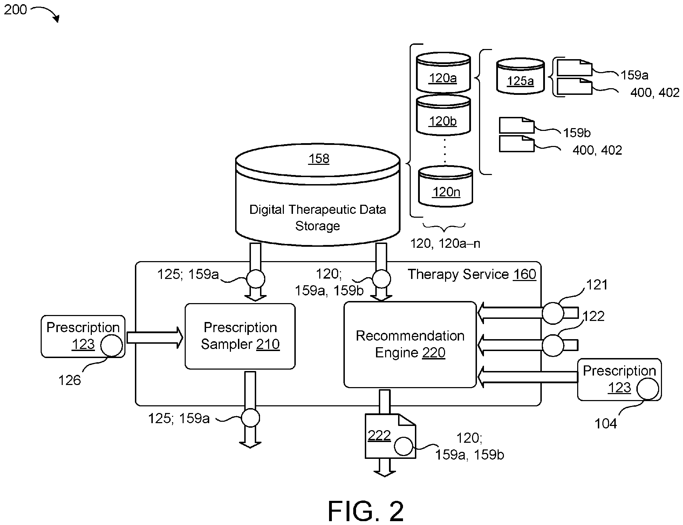

In some examples, the system 100 includes a network 106, a patient device 102, an HCP system 140, and a therapy service 160. The network 106 provides access to cloud computing resources 150 (e.g., distributed system) that execute the therapy service 160 to provide for the performance of services on remote devices. Accordingly, the network 106 allows for interaction between patients 101 and HCPs 109 with the therapy service 160. For instance, the therapy service 160 may provide the patient 101 access to the prescription digital therapeutic 120 and receive user input 121 or event data 122 input by the patient 101 associated with the patient's 101 interaction with the prescription digital therapeutics 120. In turn, the therapy service 160 may store the event data 122 on a storage resource 156. Additionally, to avoid delays in administering treatment to the patient 101 during the processing of a prescription 123 (see FIG. 2), the therapy service 160 may provision a sample prescription digital therapeutic 125 and provide the patient 101 access to the sample prescription digital therapeutic 125. Here, the therapy service 160 may receive user input 121 or event data 122 input by the patient 101 associated with the patient's 101 interaction with the sample prescription digital therapeutic 125 and augment the full prescription digital therapeutic 120 based on the user input 121 with the sample prescription digital therapeutic 125.

The network 106 may include any type of network that allows sending and receiving communication signals, such as a wireless telecommunication network, a cellular telephone network, a time division multiple access (TDMA) network, a code division multiple access (CDMA) network, Global system for mobile communications (GSM), a third generation (3G) network, fourth generation (4G) network, fifth generation (5G) network, a satellite communications network, and other communication networks. The network 106 may include one or more of a Wide Area Network (WAN), a Local Area Network (LAN), and a Personal Area Network (PAN). In some examples, the network 106 includes a combination of data networks, telecommunication networks, and a combination of data and telecommunication networks. The patient device 102, the HCP system 140, and the therapy service 160 communicate with each other by sending and receiving signals (wired or wireless) via the network 106, which, in some examples, may utilize Bluetooth, Wi-Fi, etc. In some examples, the network 106 provides access to cloud computing resources, which may be elastic/on-demand computing and/or storage resources 156 available over the network 106. The term "cloud" services generally refer to a service delivered from one or more remote devices accessible via one or more networks 106, rather than a service performed locally on a user's device.

The patient device 102 may include, but is not limited to, a portable electronic device (e.g., smartphone, cellular phone, personal digital assistant, laptop computer, or wireless tablet device), a desktop computer, or any other electronic device capable of sending and receiving information via the network 106. The patient device 102 includes data processing hardware 112 (a computing device that executes instructions), memory hardware 114, a display 116, and one or more input devices 115 in communication with the data processing hardware 112. In some implementations, the patient device includes a plurality of sensors 124, 124a-n configured to capture user data in addition to the user input 121 received by the therapy service 160. The plurality of sensors 124 may include first sensor(s) 124a, such as an accelerometer, proximity sensor, an activity or exercise monitor, location sensors such as a global positioning system (GPS), etc., to provide data about the patient 101 and/or the patient's 101 interaction with the patient device 102. Additionally or alternatively, the patient 101 may be connected to second sensor(s) 124b, such as a heart rate sensor or monitor, blood pressure sensor or monitor, a sleep sensor, an activity monitor (e.g., an electrodermal activity monitor), a skin temperature sensor, a sweat monitor, and/or any other suitable sensors or monitors (e.g., wearable sensors or monitors) in communication with the patient device 102. In some implementations, the patient 101 and the patient device 102 may be in communication with an administration unit 128, such as a delivery pump, an injection unit, an implant, an oral absorption unit (e.g., a sublingually dissolvable film or pill), an inhaler, a nasal injector, other transmucosal administration units, etc. For example, the patient device 102 may transmit a recommendation and/or an instruction to the administration unit 128 for a dosage of medication the patient 101 should take. In some examples, the patient device 102 includes the input device, e.g., a keyboard, mouse, microphones, touch sensor behind the display 116, and/or a camera for allowing the patient 101 to input data. The patient device 102 also executes, for display on the display 116 in communication with the data processing hardware 112, a graphical user interface (GUI) 400 configured to capture the user inputs 121 from the patient 101 via the input device(s) 115 (e.g., one or more of a touchscreen, a speech input, an eye gaze input, a gesture, a mouse, trackpad, stylist, etc.) for controlling functionality of the patient device 102. The GUI 400 may be an interface associated with the patient platform 103 executing on the patient device 102 that presents a plurality of objects/elements in the GUI 400. The patient device 102 may further include, or be in communication with, an audio output device (e.g., a speaker) that may output audio data to the patient 101. For instance, audible alerts may be output by the speaker to notify the patient 101 about some time sensitive event associated with the one or more prescription digital therapeutics 120. The patient device 102 may also include a physical button disposed on the patient device 102 configured to receive a tactile selection by the patient 101 for invoking the prescription digital therapeutic 120.

In some implementations, the patient device 102 executes the patient platform 103 (or accesses a web-based patient application) for establishing a connection with the therapy service 160 to access the one or more prescription digital therapeutics 120 and/or the sample prescription digital therapeutics 125 in response to a prescription 123 being issued for a prescription digital therapeutic 120. For instance, the patient 101 may have access to the patient platform 103 for a first duration (e.g., 45 days) of a sample digital prescription therapeutic 125 after the prescription 123 is issued for the full digital prescription therapeutic 120, and for a second duration (e.g., 3 months) of the full prescription digital therapeutic 120 prescribed to the patient 101 once the full prescription 123 is approved. Here, the patient device 102 may launch the patient platform 103 by initially providing a sample access code 126 when the prescription digital therapeutic 120 is prescribed by the HCP 109, the sample access code 126 allowing the patient 101 to access content associated with a sample prescription digital therapeutic 125 provisioned from the prescription digital therapeutic 120 from the therapy service 160.

The content of the sample prescription digital therapeutic 125 may be specifically tailored for treating/addressing one or more symptoms associated with the one or more specific indications that the patient 101 may be experiencing, but may include certain restrictions on content and/or time that are not included in the full prescription digital therapeutic 120. A full access code 104 allowing the patient 101 to access content associated with the full prescription digital therapeutic 120 from the therapy service 160 may be issued once the prescription 123 is approved. Approval may require signoff by a payor (i.e., an insurer), processing to establish the patient 101 in the therapy prescription system 100, and enrolling the patient 101. Additionally or alternatively, approval for the full prescription digital therapeutic 120 may require action by the HCP 109 upon a determination that the patient 101 should be prescribed the full prescription digital therapeutic 120. For example, the HCP 109 may prescribe the sample prescription digital therapeutic 125 to the patient 101 as a trial to monitor how the patient 101 interacts with the sample prescription digital therapeutic 125 and/or how the patient 101 reacts to determine whether the patient 101 should be prescribed the full prescription digital therapeutic 120.

In some implementations, after a threshold amount of time has lapsed (e.g., 45 days), the sample access code 126 for the sample prescription digital therapeutic 125 may expire, and the patient platform 103 may require the patient 101 enter the full access code 104 for the prescription digital therapeutic 120 to continue to administer treatment to the patient 101. In some implementations, the patient platform 103 prompts the patient 101 to enter the full access code 104 before the sample access code 126 expires. For example, the patient platform 103 may prompt the patient 101 to enter the full access code 104 for access to the full prescription digital therapeutic 120 two (2) days before the sample access code 104 expires. If the patient 101 enters the full access code 104, the patient platform 103 may administer, from the therapy service 160, the full prescription digital therapeutic 120. If, however, the patient 101 fails to enter the full access code 104 (e.g., the prescription 123 is not approved), the patient platform 103 may cease administration of the sample prescription digital therapeutic 125 when the sample access code 126 expires.

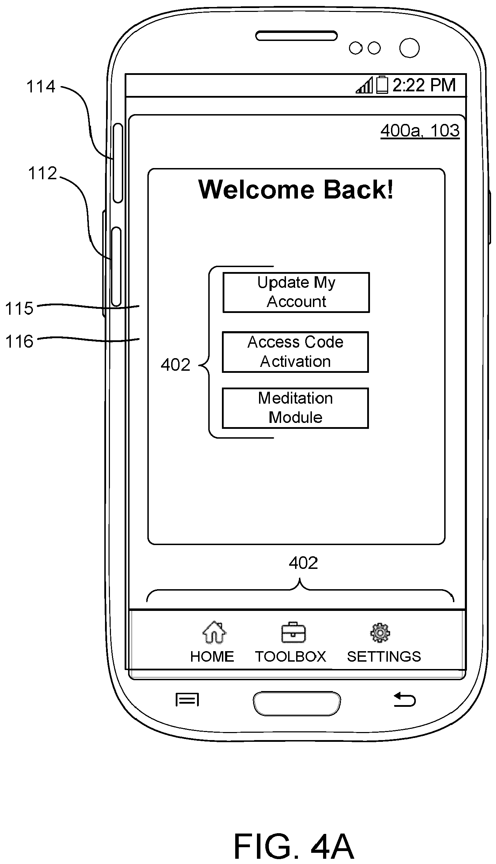

The patient platform 103, when executing on the data processing hardware 112 of the patient device 102, is configured to display a variety of GUIs 400 (e.g., the GUIs 400a-d in FIGS. 4A-4D) on the display 116 of the patient device 102 that, among other things, allow the patient 101 to (i) input event data 122 describing one or more parameters associated with the patient 101 (e.g., a signal of how the patient 101 is feeling (e.g., experiencing stress, menstruation, fatigue, sleep-deprivation, etc.)); (ii) solicit information from the patient 101; (iii) deliver therapeutic content (e.g., cognitive behavioral therapy (CBT) content) to the patient 101; (iv) allow the patient 101 to contact their HCP 109; (v) allow the patient 101 to review their progress adhering to their prescription regimen with respect to the prescription digital therapeutic 120 and/or any prescribed medication; and/or (vi) present journal entries for the patient 101 to view and/or edit. According to some examples, the therapeutic content may include textual, auditory, visual, and/or audio/visual content configured to treat one or more symptoms associated with the one or more specific indications that the patient 101 may be experiencing.

As shown in FIGS. 1 and 2, the storage resources 156 may provide a digital therapeutic database 158 (also referred to as data storage 158 and digital therapeutic data storage 158) for storing the user input 121 and the event data 122 received from the patient 101 in a corresponding patient record 105 as well as the prescription digital therapeutics 120 each including respective sample prescription digital therapeutics 125 available to the HCP 109 to prescribe for the patient 101. The digital therapeutic database 158 may be a headless content management system (CMS) that stores the prescription digital therapeutics 120 together to simplify the design, development, quality, support, and maintenance costs of the prescription digital therapeutics 120. Each of the prescription digital therapeutics 120 may include the sample prescription digital therapeutic 125 that includes the digital therapeutic content 159, 159a-n, and corresponding GUIs 400 configured to display graphical elements 402 that the patient 101 may select via user inputs 121 such as touch inputs, speech inputs, eye gaze inputs, gesture inputs, or other input techniques such as via a mouse, stylus, or keyboard. Each of the sample prescription digital therapeutics 125 in the digital therapeutic database 158 may include digital therapeutic content 159a and corresponding GUIs 400 (FIG. 2) that may overlap with, or be separate from, the digital therapeutic content 159b and corresponding GUIs 400 in the full prescription digital therapeutic 120. As such, the sample prescription digital therapeutic 125 may deliver the digital therapeutic content 159 and the corresponding GUIs 400 in a manner that is tailored to administer treatment to the patient 101 for one or more specific indications that the patient 101 may be experiencing while the patient 101 waits for the full prescription digital therapeutic 120 to be processed/approved.

In some implementations, the digital therapeutic database 158 includes third-party information (e.g., from medical databases, from the HCP, from peer-reviewed articles, etc.). The third-party information may be assimilated with the digital therapeutic content 159 and delivered to the patient 101 via one or more modules in the corresponding GUIs 400 to treat the indications that the patient 101 may be experiencing. For example, assimilating the third-party information may include distilling the substantive material from the third-party information and modifying the formatting, appearance, presentation, etc., of the substantive material so that it conforms to pre-defined standards of the prescription digital therapeutic 120. That is, the third-party information may be assimilated so that the patient 101 is presented with a combination of at least one of the third-party information and other information. In some implementations, the prescription digital therapeutics 120 include indication-specific digital therapeutics 120 and indication-agnostic digital therapeutics 120. In these implementations, the indication-agnostic digital therapeutics 120 include corresponding GUIs 400, but do not include indication-specific digital therapeutic content 159.

The patient record 105 may be encrypted while stored on the data storage 158 so that any identifying information of the patient 101 is anonymized, but may later be decrypted when the patient 101 or supervising HCP 109 requests the patient record 105 (assuming the requester is authorized/authenticated to access the patient record 105). All data transmitted over the network 106 between the patient device 102 and the cloud computing system 150 may be encrypted and sent over secure communication channels. For instance, the patient platform 103 may encrypt the user inputs 121 and the event data 122 before transmitting to the therapy service 160 via the HTTPS protocol and decrypt a patient record 105 received from the therapy service 160. When network connectivity is not available, the patient platform 103 may store the user inputs 121 and the event data 122 in an encrypted queue within the memory hardware 114 until network connectivity is available.

The HCP system 140 may be located at a clinic, doctor's office, or facility administered by the HCP 109 and includes data processing hardware 142, memory hardware 144, and a display 146. The memory hardware 144 and the display 146 are in communication with the data processing hardware 142. For instance, the data processing hardware 142 may reside on a desktop computer or portable electronic device for allowing the HCP 109 to input and retrieve data to and from the therapy service 160. In some examples, the HCP 109 may initially onboard some or all of patient data 107 at the time of prescribing the prescription digital therapeutic 120 to the patient 101. The HCP system 140 includes a keyboard 148, mouse, microphones, speakers and/or a camera.

In some implementations, the HCP system 140 (i.e., via the data processing hardware 142) executes the HCP application 110 (or accesses a web-based patient application) for establishing a connection with the therapy service 160 to input and retrieve data therefrom. For instance, the HCP system 140 may be able to access the anonymized patient record 105 securely stored by the therapy service 160 on the storage resources 156 by providing an authentication token 108 validating that the HCP 109 is supervising the patient 101 and authorized to access the corresponding patient record 105. The authentication token 108 may identify the particular patient 101 associated with the patient record 105 that the HCP system 140 is permitted to obtain from the therapy service 160. The patient record 105 may include timestamps with the event data 122 indicating the patient's interaction with the prescription digital therapeutics 120 through the patient platform 103 executing on the patient device 102. The HCP application 110, when executing on the data processing hardware 142 of the HCP system 140, is configured to display a variety of graphical user interfaces (GUIs) on the display 146 of the HCP system 140 that, among other things, allow the HCP 109 to input event data 122 describing one or more parameters associated with the patient 101, solicit information from the patient 101, and input clinical notes associated with the patient 101.

In some implementations, the HCP application 110 is in communication with a patient platform 103 for a single patient 101 and manages data associated with the patient platform 103 while simultaneously managing data associated with each of one or more prescription digital therapeutics 120 in the patient platform 103. In other implementations, the HCP application 110 is in communication with several platforms 103 associated with several patients 101, and the HCP application 110 may manage and display the data associated with the several platforms 103 for each patient 101 in the several patients 101 in any suitable manner, e.g., by toggling between different views and/or displaying certain data simultaneously. In these implementations, the data from the several platforms 103 may be displayed simultaneously in any suitable manner or the data from each patient platform 103 may be displayed discretely such that the HCP 109 is able to toggle between the discretely displayed data for the several patients 101.

The cloud computing resources 150 may be a distributed system (e.g., remote environment) having scalable/elastic resources 152. The resources 152 include computing resources 154 (e.g., data processing hardware) and/or the storage resources 156 (e.g., memory hardware). The cloud computing resources 150 execute the therapy service 160 for facilitating communications with the patient device 102 and the HCP system 140 and storing data on the storage resources 156 within the data storage 158. In some examples, the therapy service 160 and the data storage 158 reside on a standalone computing device. The therapy service 160 may provide the patient 101 with the patient platform 103 (e.g., a mobile application, a web-site application, or a downloadable program that includes a set of instructions) executable on the data processing hardware 112 and accessible through the network 106 via the patient device 102 when the patient 101 provides a valid access code 104. Similarly, the therapy service 160 may provide the HCP 109 with the HCP application 110 (e.g., a mobile application, a web-site application, or a downloadable program that includes a set of instructions) executable on the data processing hardware 142 and accessible through the network 106 via the HCP system 140.

Referring to FIG. 2, the therapy service 160 includes a prescription sampler 210 that provisions a respective sample prescription digital therapeutic 125 of a full prescription digital therapeutic 120 for administration to a patient 101. That is, the prescription sampler 210 controls the therapeutic content 159 and corresponding GUIs 400 that are presented as available options to the patient 101 while the patient 101 has access to the sample prescription digital therapeutic 125 via the sample access code 126. Advantageously, administering the sample prescription digital therapeutic 125 bridges the gap between the patient visit with the HCP 109 where the prescription 123 is written, and when the prescription 123 is processed/approved to ensure treatment is not delayed, while also offering the patient 101 with a preliminary opportunity to engage with the prescription digital therapeutic 120. Moreover, the patient platform 103 may, based on the engagement and interaction between the patient 101 and the sample prescription digital therapeutic 125, augment the full prescription digital therapeutic 120 before administering the full prescription digital therapeutic 120 to the patient 101. As just one example, if the sample prescription digital therapeutic 125 indicates that a specific module is particularly effective at treating symptoms experienced by the patient 101, then the full prescription digital therapeutic 120 may be augmented to emphasize this module or similar types of modules. As another example, if the sample prescription digital therapeutic 125 indicates that the patient 101 prefers to interact with textual (e.g., as opposed to audio-visual) modules, then the full prescription digital therapeutic 120 may be augmented to change any audio-visual modules to text-based modules.

As shown, the prescription sampler 210 receives the prescription 123 issued for a full prescription digital therapeutic 120 to treat symptoms associated with an indication experienced by a patient 101. Here, the prescription 123 includes a sample access code 126 for accessing a sample prescription digital therapeutic 125 of the full prescription digital therapeutic 120. The prescription sampler 210 may be configured to provision the sample prescription digital therapeutic 125 of the full prescription digital therapeutic 120 from the digital therapeutic data store 158 based on the sample access code 126, and deliver the sample prescription digital therapeutic 125 including the digital therapeutic content 159b and corresponding GUIs 400 to the patient platform 103. In response, the patient platform 103 of the user device 102 administers the sample prescription digital therapeutic 125 via the GUI 400 to treat the indication experienced by the user 101. As discussed above with respect to the digital therapeutic data store 158, the sample prescription digital therapeutic 125 may include certain restrictions on the digital therapeutic content 159 that is included, which may be different than the digital therapeutic content 159 available in the full prescription digital therapeutic 120.

The therapy service 160 may further include a recommendation engine 220 configured to obtain at least one of the user inputs 121 and the event data 122 indicating the patient 101 has engaged with the sample prescription digital therapeutic 125, and analyze the user input data 121 and/or the event data 122 to determine a recommendation 222 for the patient 101. The HCP 109 may use this recommendation 222 to determine whether the patient 101 should transition from the sample prescription digital therapeutic 125 to the full prescription digital therapeutic 120. For example, the recommendation 222 may include a recommendation to fill the prescription 123 for the full prescription digital therapeutic 120 based on the at least one of the user inputs 121 and the event data 122 indicating that the patient 101 has benefitted from the sample prescription digital therapeutic 125 and would respond well to the full prescription digital therapeutic 120. In these examples, the recommendation 222 generated by the recommendation engine 220 may include the digital therapeutic content 159a and corresponding GUIs, such that the prescription digital platform 103 is transitioned to include the full prescription digital therapeutic 120 with the digital therapeutic content 159a, 159b and corresponding GUIs 400. The recommendation engine 220 may further require a valid full access code 104 indicating that the subscription 123 is approved/processed completely before generating the recommendation 222 to transition the prescription digital therapeutic platform 103 to include the full prescription digital therapeutic 120.

Conversely, the recommendation 222 generated by the recommendation engine 220 may include a recommendation to the HCP 109 and/or the patient 101 to rescind (and/or not fill) the prescription 123 for the full prescription digital therapeutic 120 based on the at least one of the user inputs 121 and the event data 122. Here, the recommendation engine 220 may analyze the at least one of the user inputs 121 and the event data 122 and determine that the patient 101 has not benefitted from the sample prescription digital therapeutic 125, and therefore would not benefit from continued usage of the prescription digital therapeutic 120.

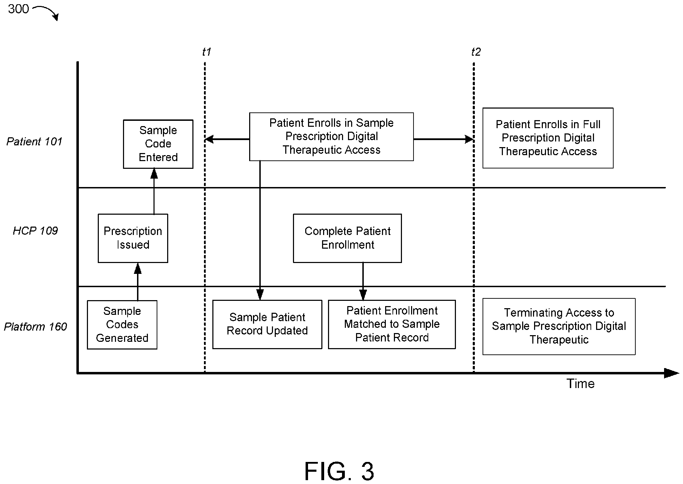

Referring to FIG. 3, schematic view 300 shows the processes carried out by the patient 101, the HCP 109, and the Platform 160 set on an x-axis representing a duration of time. Here, the platform 160 may first generate a sample access code 126 for access to a sample prescription digital therapeutic 125 of full prescription digital therapeutic 120. The HPC 109 may receive the sample access code 126 to provide along with an issued prescription 123, where the patient 101 may enter the sample access code 126 and begin enrollment immediately. As indicated by *t1,* the patient 101 enrolls in access to the sample prescription digital therapeutic 125, where the sample prescription digital therapeutic 125 remains valid until *t2*. While the patient 101 is enrolled in, and using the sample prescription digital therapeutic 125, the platform 160 receives an update that the patient 101 has enrolled, and after the HCP 109 completes the patient enrollment, matches the patient enrollment from the HCP 109 to the patient record for the patient 101 using the sample prescription digital therapeutic 125. As indicated at time *t2,* after the sample access has expired, the platform 160 terminates access to the sample prescription digital therapeutic 125. As such, if the patient 101 is enrolled in access to the full prescription digital therapeutic 120 (e.g., via a full access code 104), the patient 101 continues to use the prescription digital therapeutic. In some implementations, the patient 101 is prompted to enter a full access code 104 prior to the sample prescription digital therapeutic 125 access expiring.

Referring to FIGS. 4A-4D, schematic views of exemplary GUIs 400a-d of the prescription digital therapeutic 120 are (e.g., by execution of the prescription digital therapeutic platform 103) displayed on the display 116 of the patient device 102 for treating symptoms associated with one or more indications. The example GUIs 400a-d are configured to display the digital therapeutic content 159 including one or more graphical elements 402 that the patient 101 may select via the input device(s) 115. As discussed above with respect to FIG. 2, the digital therapeutic content 159 may be delivered in the sample prescription digital therapeutic 125, in the full prescription digital therapeutic 120, or both, where the digital therapeutic content 159 is tailored to the patient 101 based on the indications that the patient 101 may be experiencing.

Referring to FIG. 4A, in some implementations, upon launching the indication-agnostic prescription digital therapeutic platform 103, the indication-agnostic prescription digital therapeutic platform 103 displays a first GUI 400a including first graphical user interface elements 402 associated with a prescription digital therapeutic 120. As shown, the GUI 400a includes a welcome screen for the patient 101 and provides graphical user interface elements 402 that allow the patient 101 to update an account of the patient 101, activate a prescription digital therapeutic 120, or access an indication-agnostic digital therapeutic 120 such as a meditation module. The first graphical user interface elements 402 may further include elements for the patient 101 to navigate within the prescription digital therapeutic platform 103 (i.e., between one or more prescription digital therapeutics 120).

In some examples, the HCP 109 issues a prescription 123 for a prescription digital therapeutic 120. As discussed with respect to FIG. 1 above, while in the office of the HCP 109, or shortly after leaving, the patient 101 may receive a sample access code 126 when the prescription digital therapeutic 120 is prescribed by the HCP 109. The patient may enter the sample access code 126 in the graphical user interface 402 "Access Code Activation" to access the digital therapeutic content 159 included in the sample prescription digital therapeutic 125. As discussed above with reference to FIG. 2, the sample prescription digital therapeutic 125 may include certain restrictions on the digital therapeutic content 159 and/or access time that are not included in the full prescription digital therapeutic 120. In response to the prescription 123 being issued for the prescription digital therapeutic 120, the prescription digital therapeutic platform 103 augments the GUI 400 to include the digital therapeutic content 159a associated with the sample prescription digital therapeutic 125. In other words, the prescription digital therapeutic platform 103 is augmented to include the second GUI 400b associated with the sample prescription digital therapeutic 125.

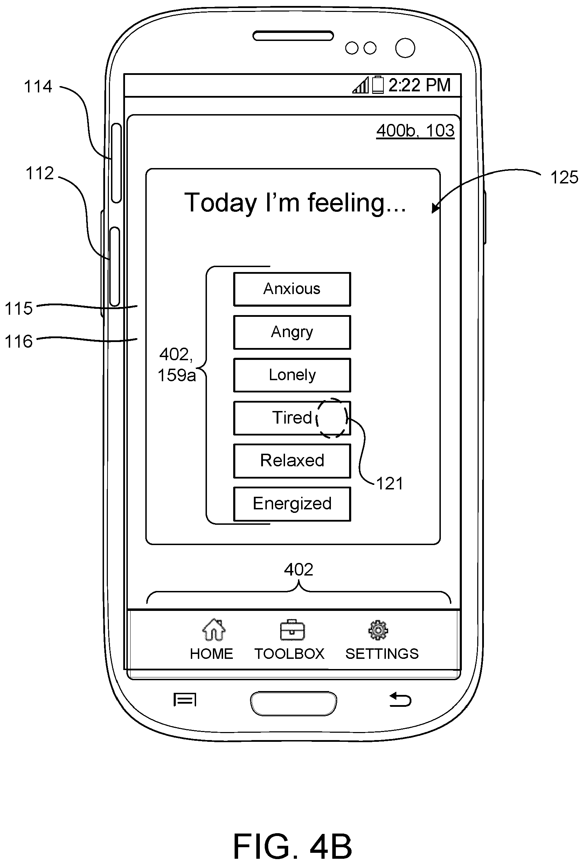

Referring now to FIG. 4B, the prescription digital therapeutic platform 103 displays the second GUI 400b of the sample prescription digital therapeutic 125. In some implementations, the prescription digital therapeutic platform 103 displays the second GUI 400b in response to the patient 101 entering the sample access code 126 in the graphical user interface element 402 of FIG. 4A. As shown, the sample prescription digital therapeutic 125 may treat symptoms associated with generalized anxiety disorder (GAD), where the prescription digital therapeutic platform 103 administers the digital therapeutic content 159a of the sample prescription digital therapeutic 125 via the second GUI 400b to treat GAD. Here, the second GUI 400b may additionally display graphical user interface elements 402 that allow the patient 101 to navigate within the digital therapeutic platform 103.

In the example shown, the second GUI 400b provides the digital therapeutic content 159a and the corresponding graphical user elements 402 for the sample prescription digital therapeutic 125, where each of the graphical user elements 402 is associated with a corresponding feeling of the patient 101. The graphical user elements 402 may be prepopulated based on common feelings a typical patient diagnosed with GAD may be experiencing. The second GUI 400b allows the patient 101 to input a particular feeling they are presently experiencing, or has recently experienced. In the example, the graphical user elements 402 include an "Anxious" element 402, an "Angry" element 402, a "Lonely" element 402, a "Tired" element 402, a "Relaxed" element 402, and an "Energized" element 402. The graphical user elements 402 do not represent an exhaustive list, but rather are an exemplary list of elements 400b that may be included as part of the sample prescription digital therapeutic 125 (via the second GUI 400b).

While administering the digital therapeutic content 159a, the patient device 102 detects a user input 121 corresponding to the "Tired" element 402 indicating the patient 101 is feeling tired. Here, the recommendation engine 220 (FIG. 2) obtains the user input 121 and analyzes the user input 121 to determine a recommendation 22 for the patient 101. As discussed above, the recommendation 222 may be provided to the HCP 109 as input on whether to continue the course of treatment (via the prescription digital therapeutic 120) for the patient 101. In response to determining a recommendation 222 for the patient 101, the prescription digital therapeutic platform 103 is augmented to include a prompt to the patient 101 via a third GUI 400c to access the full prescription digital therapeutic 120.

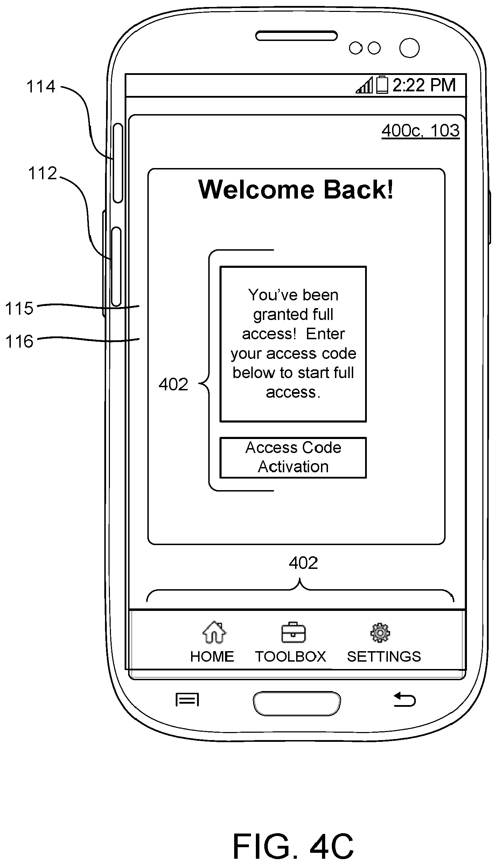

In the example shown in FIG. 4C, the prescription digital therapeutic platform 103 generates a prompt in the GUI 400c including the graphical element 402 "You've been granted full access! Enter your access code below to start full access." and the graphical element 402 "Access Code Activation." to access the digital therapeutic content 159 included in the full prescription digital therapeutic 120. As discussed above with reference to FIG. 2, the full prescription digital therapeutic 120 may include full/unrestricted access to the digital therapeutic content 159. In response to the prescription 123 being processed/approved for the prescription digital therapeutic 120, the prescription digital therapeutic platform 103 augments the GUI 400 to include the prescription digital content 159b associated with the full prescription digital therapeutic 120 in addition to the prescription digital content 159a associated with the sample prescription digital therapeutic 125. In other words, the prescription digital therapeutic platform 103 is augmented to include the fourth GUI 400d associated with the full prescription digital therapeutic 120, and to administer the full prescription digital therapeutic 120 to the patient 101 via the GUI 400d.

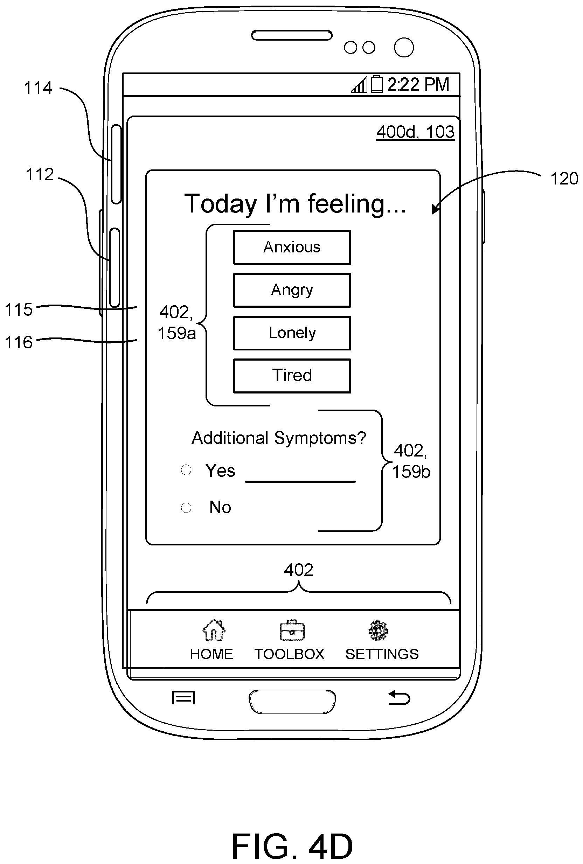

Referring now to FIG. 4D, the prescription digital therapeutic platform 103 displays the GUI 400d of the full prescription digital therapeutic 120. In some implementations, the prescription digital therapeutic platform 103 displays the GUI 400d in response to the patient 101 entering the full access code 104 in the graphical user interface element 402 of FIG. 4C. As shown, the full prescription digital therapeutic 120 may include digital therapeutic content 159a of the sample prescription digital therapeutic 125 as well as the digital therapeutic content 159b. Here, the digital therapeutic content 159b of the full prescription digital therapeutic 120 may include the additional feature of allowing the patient 101 to enter "Additional Symptoms" not included in the list of symptoms in the graphical user elements 402 associated with the digital therapeutic content 159a. Additionally, the digital therapeutic content 159b associated with the sample prescription digital therapeutic 125 may be modified in the full prescription digital therapeutic 120 based on the patient device 102 detecting a user input 121 corresponding to the "Tired" element 402 (FIG. 4B) indicating the patient 101 is feeling tired. As shown in FIG. 4D, the graphical user elements 402 in the digital therapeutic content 159b is more closely tailored to GAD based on the interactions between patient 101 and the sample prescription digital therapeutic 125.

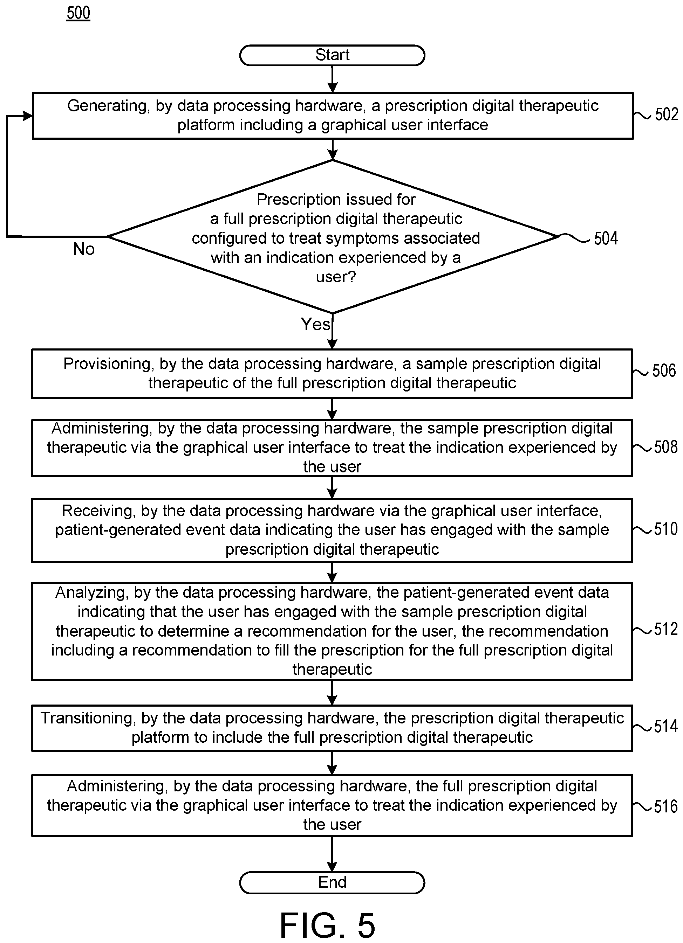

Referring to FIG. 5, a flowchart illustrating a method 500 for implementing the prescription digital therapeutic 120 including a sample prescription digital therapeutic 125 is generally shown. The method 500 may include a treatment of at least one indication. The method 500 includes, at operation 502, generating, by data processing hardware 610, a prescription digital therapeutic platform 103 including a graphical user interface (GUI) 400. At operation 504, the method 500 includes determining whether a prescription 123 has been issued for a full prescription digital therapeutic 120 configured to treat symptoms associated with an indication experienced by a user 101. If the method 500 determines, at operation 504, that a prescription 123 has not been issued for a full prescription digital therapeutic 120, then the method 500 returns to operation 502, where the treatment may be continued. If the method 500 determines, at 504, that a prescription 123 has been issued for a full prescription digital therapeutic 120, then the method 500 proceeds to operation 506.

At operation 506, the method 500 includes, in response to the prescription 123 being issued for the full prescription digital therapeutic 120, provisioning, by the data processing hardware 610, a sample prescription digital therapeutic 125 of the full prescription digital therapeutic 120. The method 500 also includes, at operation 508, administering, by the data processing hardware 610, the sample prescription digital therapeutic 125 via the GUI 400 to treat the indication experienced by the user 101. The method 500 also includes, at operation 510, receiving, by the data processing hardware 610, via the GUI 400, patient-generated event data 122 indicating the user 101 has engaged with the sample prescription digital therapeutic 125.

At operation 512, the method 500 further includes analyzing, by the data processing hardware 610, the patient-generated event data 122 indicating that the user 101 has engaged with the sample prescription digital therapeutic 120 to determine a recommendation 222 for the user 101. Here, the recommendation 222 may include a recommendation to fill the prescription 123 for the full prescription digital therapeutic 120. In response to determining the recommendation 222 for the user 101, the method 500 also includes, at operation 514, transitioning, by the data processing hardware 610, the prescription digital therapeutic platform 103 to include the full prescription digital therapeutic 120. At operation 516, the method 500 also includes administering, by the data processing hardware 610, the full prescription digital therapeutic 120 via the GUI 400 to treat the indication experienced by the user 101. It should be understood that the method 500 may include additional or fewer steps than those shown and described, and certain steps may be omitted or performed in any suitable order.

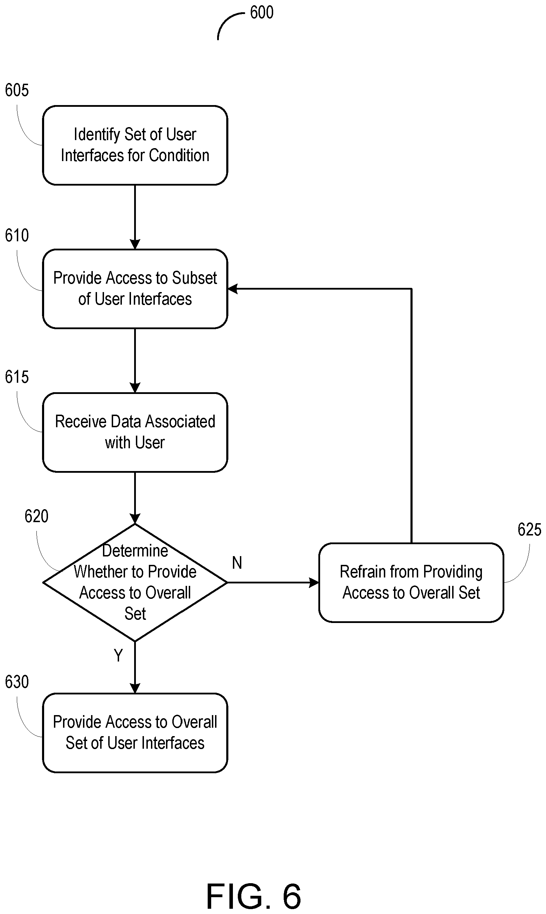

Referring now to FIG. 6, depicted is a flow diagram of a process 600 for controlling access to user interfaces. The process 600 can be implemented or performed by any of the components detailed herein, such as the data processing hardware 112, 142, 154 or a computing device 700, among others. The process 600 can include any of the operations detailed herein. In brief overview, under the process 600, a computing system can identify a set of user interfaces for a condition (605). The computing system can provide access to a subset of user interfaces (610). The computing system can receive data associated with a user (615). The computing system can determine whether to provide access to the overall set of user interfaces (620). If the determination is to not provide access, the computing system can refrain from providing access to the overall set (630). Otherwise, if the determination is to provide access, the computing system can provide access to the overall set of user interfaces (625).

In further detail, a computing system (e.g., the data processing hardware 112, 142, 154, the therapy service 160, or the computing device 700) can obtain, select, or otherwise identify a set of user interfaces (e.g., GUIs 400) for a condition (605). The set of user interfaces can provide or present at least a portion of digital therapeutic content (e.g., the digital therapeutic content 159) to a user (e.g., the patient 101). The digital therapeutic content can be used to treat, alleviate, or otherwise address a condition via interactions with one or more of the set of user interfaces. At least a subset of user interfaces can correspond to a sample digital therapeutic (e.g., the sample prescription digital therapeutic 125) to present a corresponding portion of the digital therapeutic content to the user. In some embodiments, the subset of user interfaces can correspond to an indication-agnostic digital therapeutic. The overall set of user interfaces can correspond to a full digital therapeutic (e.g., the full prescription digital therapeutics 120) to present the entirety of the digital therapeutic content to the user. In some embodiments, the overall set of user interfaces can correspond to an indication-specific digital therapeutic.

The digital therapeutic content can be associated with the digital therapeutic (e.g., sample or full digital therapeutic) to be provided to the user to address the condition (sometimes herein referred to as medical condition or indication). The condition can include, for example, at least one of: substance use disorder, opioid use disorder, chronic insomnia, alcohol use disorder, schizophrenia, generalized anxiety disorder, major depressive disorder, bipolar, posttraumatic stress disorder, acute and chronic pain, migraine, multiple sclerosis, epilepsy, irritable bowel syndrome, specialty gastroenterology, cancer, or cardiovascular disease. In at least partial concurrence with the digital therapeutic content (e.g., the sample or full digital therapeutic provided via the user interfaces), the user may be on a medication (e.g., traditional drug therapy) to address the condition.

To identify, the computing system can retrieve, obtain, or otherwise receive an indication (e.g., the prescription 123) to provide the user with at least a portion of the digital therapeutic (e.g., the sample or full prescription digital therapeutic) from a remote service (e.g., the HCP 109). In some embodiments, the computing system can receive the indication from the user device associated with the user. The indication can correspond to a prescription issued by an entity associated with the remote service. The indication can reference or identify the condition to be addressed in the user. In some embodiments, the indication can identify or include an access code (e.g., the sample access code 126) for accessing at least a portion of the digital therapeutic. With the receipt of the indication, the computing system can access a database (e.g., the digital therapeutic data storage 158) to find, select, or identify the set of user interfaces for the digital therapeutic. In some embodiments, the computing system can generate the set of user interfaces for the digital therapeutic.

The computing system can grant, permit, or otherwise provide access to a user device (e.g., the patient device 102) of the user to a subset of user interfaces (610). The subset of user interfaces can correspond to the sample prescription digital therapeutic. The subset of user interfaces can provide or present a corresponding portion of the digital therapeutic content to the user. The provision of access to the sample prescription digital therapeutic may be for a time window. In some embodiments, the computing device can provide access to at least one user interface different from the overall set of user interfaces for the full prescription digital therapeutic. For example, the at least one user interface for the sample prescription digital therapeutic can overlap or be separate from the set of user interfaces for the full prescription digital therapeutic. In some embodiments, the computing system can restrict the user device access to a remainder of the overall set of user interfaces, until receipt of further indication.

In some embodiments, to provide access, the computing system can extract or identify the access code from the indication (e.g., issued as the prescription 123). With the identification of the sample code, the computing system can determine to provide the user device associated with the user access to the subset of user interfaces for the sample prescription digital therapeutic. In some embodiments, the computing system (or the remote system associated with the HCP 109) can produce, output, or otherwise generate the access code for accessing to the subset of user interfaces. With the generation of the access code, the computing system can send, transmit, or otherwise provide the access code, upon receipt of the indication for provision of the digital therapeutic (e.g., upon receipt of the prescription from the HCP 109). The computing system can provide the user device access to the subset of user interfaces for the sample prescription digital therapeutics, in response to input or entry of the access code via the user device. For example, subsequent to providing the access code, the user can input the access code on an application (e.g., the patient platform 103) on the user device to gain access to the subset of user interfaces.

With the provision of access, the computing system can present or provide one or more of the subset of user interfaces via a display (e.g., the display 116) of the user device. For example, upon request, the computing system can provide at least one of the subset of user interfaces for the sample digital therapeutic. In some embodiments, upon providing access, the computing system can maintain a timer to keep track of a time elapsed since the provision of access to the sample prescription digital therapeutic or the provision of the access token. The computing system can compare the elapsed time with the time window (or threshold time) for the provision of access to the sample prescription digital therapeutic. If the elapsed time exceeds the time window, the computing system can identify or determine that the time window has expired. Upon determination of the expiration of the time window, the computing system can lock or restrict access to the subset of user interfaces for the sample prescription digital therapeutic. Otherwise, if the elapsed time does not exceed the time window, the computing system can continue to keep track of the elapsed time. The computing system can also continue provision of the access to the subset of user interfaces for the sample prescription digital therapeutic.

The computing system can retrieve, identify, or otherwise receive data associated with the user (615). The receipt of the data can be in at least partial concurrence with the provision of access to the subset of user interfaces for the sample prescription digital therapeutic. In some embodiments, the computing system can receive the data (e.g., the user input 121 or event data 122) from the user device. The data can identify interactions by the user with the one or more of the subset of user interfaces for the sample prescription digital therapeutic. In some embodiments, the computing system can identify or select one or more user interfaces (e.g., the GUIs 400) to add to the subset of user interfaces based on the data identifying the interactions. The addition can be during the time window for the sample prescription digital therapeutic. For example, the computing system can augment the subset of user interfaces for the sample digital therapeutic based on a user state indicated in the interaction data received. With the selection, the computing system can provide the user device access to the one or more selected user interfaces, in addition to the subset of user interfaces for the sample digital therapeutic.

The computing system can identify or determine whether to grant, permit, or otherwise provide the user device with access to the overall set of user interfaces (620). In some embodiments, the computing system can determine whether to provide access to the entirety of the digital therapeutic content based on the data identifying the interaction with the subset of user interfaces for the corresponding portion of the digital therapeutic content. In determining, the computing system can identify or determine whether the interactions by the user with the subset of user interfaces for the sample digital therapeutic satisfy a criterion within the time window for the sample prescription digital therapeutic. The criterion can identify or define one or more conditions for the corresponding portion of the digital therapeutic content, under which the user is to be provided access to the full prescription digital therapeutic. For example, the criterion can specify that the user is to be provided the full prescription digital therapeutic, if the sample prescription digital therapeutic was effective at addressing symptoms associated with the condition or if the rate of engagement (e.g., measured via interactions) satisfies a threshold rate.

When the interactions identified in the data exceeds the threshold engagement rate, the computing system can determine that the interactions satisfy the criterion. In addition, when the data indicate that the sample prescription digital therapeutic is effective (e.g., as indicated by user input) at addressing the condition, the computing system can determine that the interactions satisfy the criterion. If the interactions satisfy the criterion, the computing system can determine that the user is to be provided access to the entirety of the digital therapeutic content. Conversely, when the interactions identified in the data do not exceed the threshold engagement rate, the computing system can determine that the interactions do not satisfy the criterion. In addition, when the data indicate that the sample prescription digital therapeutic is not effective in a specific user at addressing the condition, the computing system can determine that the interactions do not satisfy the criterion. If the interactions do not the criterion, the computing system can determine that the user is not to be provided access to the entirety of the digital therapeutic content. In some embodiments, the computing system may modify the digital therapeutic content, based on the user interactions, to render the digital therapeutic content more effective for the specific user.

In some embodiments, the computing system can determine whether to provide access by waiting for receipt of an indication to provide access to the entirety of digital therapeutic (e.g., the full prescription digital therapeutic) from the remote service (e.g., HCP 109). In some embodiments, the computing system can retrieve, identify, or otherwise receive an indication to provide access to the entirety of digital therapeutic (e.g., the full prescription digital therapeutic) from the remote service (e.g., the HCP 109). In some embodiments, the computing system can receive the indication, in response to an approval by the remote service of the provision of the entirety of the digital therapeutic content to the user. For example, after issuing the prescription for the sample digital therapeutic, the entity associated with the remote service can enroll the user for the full prescription digital therapeutic upon receipt of approval from an insurer for the user. Upon enrollment, the remote service can send the indication of the approval for the full prescription digital therapeutic to the computing system. When the indication is received, the computing system can determine that the user is to be provided access to the overall set of user interfaces. Conversely, when the indication is not received (e.g., within the time window for the sample digital therapeutic), the computing system can determine that the user is not to be provided access to the overall set of user interfaces.

If the determination is to not provide access, the computing system can refrain from providing access to the overall set of user interfaces (630). In some embodiments, the computing system can refrain from providing access to the overall set of user interfaces for the full prescription digital therapeutics, when the interactions identified in the data do not satisfy the criterion. The computing system can continue to provide the user device access to the sample prescription digital therapeutic. The computing system can repeat the process 600 from step 610.

Otherwise, if the determination is to provide access, the computing system can grant, permit, or otherwise provide access to the overall set of user interfaces (625). The overall set of user interfaces can provide or present the entirety of the digital therapeutic content (e.g., the digital therapeutic content 159) to the user. The provision of access to the full prescription digital therapeutic may be for subsequent to the time window, during which the sample prescription digital therapeutic is made available to the user. The overall set of user interfaces for the full prescription digital therapeutic can include the subset of user interfaces for the sample prescription digital therapeutic. The overall set of user interfaces can include additional user interfaces (e.g., modules) to further treat, alleviate, or otherwise address the condition in the user. In some embodiments, the computing system can provide access to the indication-specific digital therapeutic.

In some embodiments, the computing system (or the remote system associated with the HCP 109) can produce, output, or otherwise generate an access code (e.g., the full access code 140) for accessing to the overall set of user interfaces. The access code for the full prescription digital therapeutic can differ from the access code for the sample prescription digital therapeutic. With the generation of the access code, the computing system can send, transmit, or otherwise provide the access code to the user device. The computing system can provide the access code with an indication that the user is to be transitioned from the sample prescription digital therapeutic to the full prescription digital therapeutic. The computing system can provide the user device access to the overall set of user interfaces for the full prescription digital therapeutics, in response to input or entry of the access code via the user device. For example, subsequent to providing the access code, the user can input the access code on the application on the user device to gain access to the overall set of user interfaces for the full prescription digital therapeutic.

With the providing of access, the computing system can present or provide one or more of the overall set of user interfaces via the display of the user device. For example, upon request, the computing system can provide at least one of the overall set of user interfaces for the full digital therapeutic. In some embodiments, upon providing access, the computing system can maintain a timer to keep track of a time elapsed since the provision of access to the full prescription digital therapeutic or the provision of the access token. The computing system can compare the elapsed time with the time window (or threshold time) for the provision of access to the full prescription digital therapeutic. If the elapsed time exceeds the time window, the computing system can identify or determine that the time window has expired. Upon determination of the expiration of the time window, the computing system can lock or restrict access to the overall set of user interfaces for the full prescription digital therapeutic. Otherwise, if the elapsed time does not exceed the time window, the computing system can continue to keep track of the elapsed time. The computing system can also continue provision of the access to the overall set of user interfaces for the full prescription digital therapeutic.

In conjunction with the provision of access, the computing system can retrieve, identify, or otherwise receive data associated with the user. The receipt of the data can be in at least partial concurrence with the provision of access to the overall set of user interfaces for the full prescription digital therapeutic. In some embodiments, the computing system can receive the data (e.g., the user input 121 or event data 122) from the user device. The data can identify interactions by the user with the one or more of the overall set of user interfaces for the full prescription digital therapeutic. In some embodiments, the computing system can identify or select one or more user interfaces (e.g., the GUIs 400) to add to the overall set of user interfaces based on the data identifying the interactions. The addition can be during the time window for the full prescription digital therapeutic. For example, the computing system can augment the overall set of user interfaces for the full prescription digital therapeutic based on a user state indicated in the interaction data received. With the selection, the computing system can provide the user device access to the one or more selected user interfaces, in addition to the overall set of user interfaces for the full prescription digital therapeutic. In some embodiments, the computing system can modify or change at least one of the user interfaces based on the data. For instance, the computing system can change a module corresponding to one or more of the user interfaces from audio-visual modules to text-based modules.

In this manner, the computing system can regulate and control user access to one or more of a set of user interfaces for providing digital therapeutic content to address various conditions in the user. Based on data received about the user indicating engagement or efficacy of the digital therapeutic, the computing system can dynamically titrate or adjust the user interfaces available for access by the user to provide additional digital therapeutic content. By controlling the access to user interfaces in this fashion, the computing system can limit or reduce the consumption of computing resources (e.g., processing and memory) and network bandwidth, which would have been otherwise been wasted from providing the full set of user interfaces from the beginning. In addition, the computing system can restrict or limit access to certain user interfaces through a remote service, thereby expanding the functionality of other computing devices to control presentation of user interfaces on the user device (e.g., for security or efficacy purposes). Furthermore, since the user is initially provided with a sample rather than the full content, the computing system can increase the functionality and utility of the user device and improve the quality of human-computer interactions (HCI), relative to either not providing the content at all or providing the user with an overwhelming set of user interfaces.

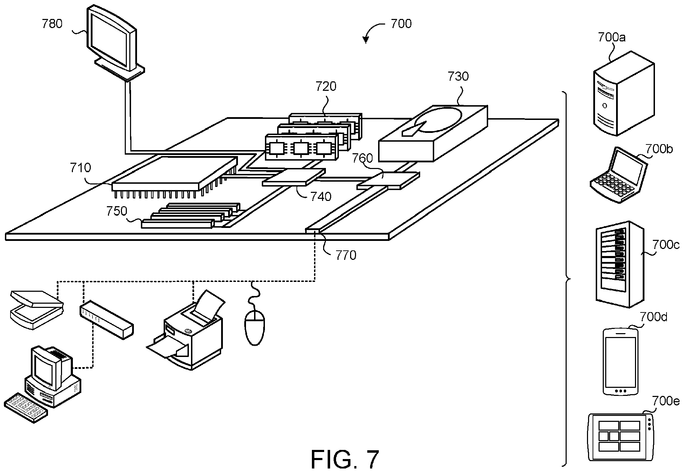

FIG. 7 is schematic view of an example computing device 700 that may be used to implement the systems and methods described in this document. The computing device 700 is intended to represent various forms of digital computers, such as laptops, desktops, workstations, personal digital assistants, servers, blade servers, mainframes, and other appropriate computers. The components shown here, their connections and relationships, and their functions, are meant to be exemplary only, and are not meant to limit implementations of the inventions described and/or claimed in this document.

The computing device 700 includes a processor 710 (e.g., data processing hardware 112, 142, 154 of FIG. 1), memory 720 (e.g., memory hardware 114, 144, 156 of FIG. 1), a storage device 730, a high-speed interface/controller 740 connecting to the memory 720 and high-speed expansion ports 750, and a low speed interface/controller 760 connecting to a low speed bus 770 and a storage device 730. Each of the components 710, 720, 730, 740, 750, and 760, are interconnected using various busses, and may be mounted on a common motherboard or in other manners as appropriate. The processor 710 can process instructions for execution within the computing device 700, including instructions stored in the memory 720 or on the storage device 730 to display graphical information for a graphical user interface (GUI) on an external input/output device, such as display 780 coupled to high speed interface 740. In other implementations, multiple processors and/or multiple buses may be used, as appropriate, along with multiple memories and types of memory. Also, multiple computing devices 700 may be connected, with each device providing portions of the necessary operations (e.g., as a server bank, a group of blade servers, or a multi-processor system).

The memory 720 stores information non-transitorily within the computing device 700. The memory 720 may be a computer-readable medium, a volatile memory unit(s), or non-volatile memory unit(s). The non-transitory memory 720 may be physical devices used to store programs (e.g., sequences of instructions) or data (e.g., program state information) on a temporary or permanent basis for use by the computing device 700. Examples of non-volatile memory include, but are not limited to, flash memory and read-only memory (ROM) / programmable read-only memory (PROM) / erasable programmable read-only memory (EPROM) / electronically erasable programmable read-only memory (EEPROM) (e.g., typically used for firmware, such as boot programs). Examples of volatile memory include, but are not limited to, random access memory (RAM), dynamic random access memory (DRAM), static random access memory (SRAM), phase change memory (PCM) as well as disks or tapes.

The storage device 730 is capable of providing mass storage for the computing device 700. In some implementations, the storage device 730 is a computer-readable medium. In various different implementations, the storage device 730 may be a floppy disk device, a hard disk device, an optical disk device, or a tape device, a flash memory or other similar solid state memory device, or an array of devices, including devices in a storage area network or other configurations. In additional implementations, a computer program product is tangibly embodied in an information carrier. The computer program product contains instructions that, when executed, perform one or more methods, such as those described above. The information carrier is a computer- or machine-readable medium, such as the memory 720, the storage device 730, or memory on processor 710.

The high speed controller 740 manages bandwidth-intensive operations for the computing device 700, while the low speed controller 760 manages lower bandwidth-intensive operations. Such allocation of duties is exemplary only. In some implementations, the high-speed controller 740 is coupled to the memory 720, the display 780 (e.g., through a graphics processor or accelerator), and to the high-speed expansion ports 750, which may accept various expansion cards (not shown). In some implementations, the low-speed controller 760 is coupled to the storage device 730 and a low-speed expansion port 790. The low-speed expansion port 790, which may include various communication ports (e.g., USB, Bluetooth, Ethernet, wireless Ethernet), may be coupled to one or more input/output devices, such as a keyboard, a pointing device, a scanner, or a networking device such as a switch or router, e.g., through a network adapter.

The computing device 700 may be implemented in a number of different forms, as shown in the figure. For example, it may be implemented as a standard server 700a or multiple times in a group of such servers 700a, as a laptop computer 700b, as part of a rack server system 700c, as a mobile device 700d (such as a smart phone), or as a tablet computer 700e.

Various implementations of the systems and techniques described herein can be realized in digital electronic and/or optical circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

A software application (i.e., a software resource) may refer to computer software that causes a computing device to perform a task. In some examples, a software application may be referred to as an "application," an "app," or a "program." Example applications include, but are not limited to, system diagnostic applications, system management applications, system maintenance applications, word processing applications, spreadsheet applications, messaging applications, media streaming applications, social networking applications, and gaming applications.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" and "computer-readable medium" refer to any computer program product, non-transitory computer readable medium, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

The non-transitory memory may be physical devices used to store programs (e.g., sequences of instructions) or data (e.g., program state information) on a temporary or permanent basis for use by a computing device. The non-transitory memory may be volatile and/or non-volatile addressable semiconductor memory. Examples of non-volatile memory include, but are not limited to, flash memory and read-only memory (ROM) / programmable read-only memory (PROM) / erasable programmable read-only memory (EPROM) / electronically erasable programmable read-only memory (EEPROM) (e.g., typically used for firmware, such as boot programs). Examples of volatile memory include, but are not limited to, random access memory (RAM), dynamic random access memory (DRAM), static random access memory (SRAM), phase change memory (PCM) as well as disks or tapes.

The processes and logic flows described in this specification can be performed by one or more programmable processors, also referred to as data processing hardware, executing one or more computer programs to perform functions by operating on input data and generating output. The processes and logic flows can also be performed by special purpose logic circuitry, e.g., an FPGA (field programmable gate array) or an ASIC (application specific integrated circuit). Processors suitable for the execution of a computer program include, by way of example, both general and special purpose microprocessors, and any one or more processors of any kind of digital computer. Generally, a processor will receive instructions and data from a read only memory or a random access memory or both. The essential elements of a computer are a processor for performing instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. However, a computer need not have such devices. Computer readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto optical disks; and CD ROM and DVD-ROM disks. The processor and the memory can be supplemented by, or incorporated in, special purpose logic circuitry.

To provide for interaction with a user, one or more aspects of the disclosure can be implemented on a computer having a display device, e.g., a CRT (cathode ray tube), LCD (liquid crystal display) monitor, or touch screen for displaying information to the user and optionally a keyboard and a pointing device, e.g., a mouse or a trackball, by which the user can provide input to the computer. Other kinds of devices can be used to provide interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's client device in response to requests received from the web browser.

A number of implementations have been described. Nevertheless, it will be understood that various modifications may be made without departing from the spirit and scope of the disclosure. Accordingly, other implementations are within the scope of the following claims.

## Claims

1. A method of controlling access to user interfaces, comprising:
identifying, by one or more processors, a plurality of user interfaces to present digital therapeutic content to a user to address a condition using one or more of the plurality of user interfaces;
providing, by the one or more processors, to a user device associated with the user over a time window, access to a subset of the plurality of user interfaces to present a corresponding portion of the digital therapeutic content to the user;
receiving, by the one or more processors, data from the user device, the data identifying interactions by the user with one or more of the subset of the plurality of user interfaces;
determining, by the one or more processors, that the interactions by the user satisfy a criterion with the corresponding portion of the digital therapeutic content within the time window, based on the data from the user device; and
providing, by the one or more processors, to the user device, access to the plurality of user interfaces to present an entirety of the digital therapeutic content to the user, responsive to determining that the interactions satisfy the criterion.

2. The method of claim 1, wherein determining that the user satisfy the criterion further comprises determining that the corresponding portion of the digital therapeutic content is effective at addressing the condition associated with the user within the time period, based on the data.

3. The method of claim 1, further comprising:
determining, by the one or more processors, that second interactions by the user do not satisfy the criterion with the corresponding portion of the digital therapeutic content within the time window, based on second data from the user device; and
refraining, by the one or more processors, from providing the user device access to the plurality of user interfaces to present the entirety of the digital therapeutic content, responsive to determining that the second interactions do not satisfy the criterion.

4. The method of claim 1, further comprising generating, by the one or more processors, an access code to provide the user device access to the plurality of user interfaces for the entirety of the digital therapeutic content, responsive to determining that the interactions satisfy the criterion, and
wherein providing the user device access to the plurality of user interfaces further comprises providing the user device access to the plurality of user interfaces, responsive to entry of the access code via the user device.

5. The method of claim 1, wherein providing the user device access to the subset of the plurality of user interfaces further comprises providing the user device access to a second user interface different from any of the plurality of user interfaces.

6. The method of claim 1, wherein the user is on a medication to address the condition, in at least partial concurrence with at least one of (i) provision of the subset of the plurality of user interfaces for the portion of the digital therapeutic content or (ii) provision of the plurality of user interfaces for the entirety of the digital therapeutic content.

7. The method of claim 1, wherein the condition comprises at least one of: substance use disorder, opioid use disorder, chronic insomnia, alcohol use disorder, schizophrenia, generalized anxiety disorder, major depressive disorder, bipolar, posttraumatic stress disorder, acute and chronic pain, migraine, multiple sclerosis, epilepsy, irritable bowel syndrome, specialty gastroenterology, cancer, or cardiovascular disease.

8. A method of controlling access to user interfaces, comprising:
identifying, by one or more processors, a plurality of user interfaces to present digital therapeutic content to a user to address a condition using one or more of the plurality of user interfaces;
providing, by the one or more processors, to a user device associated with the user, access to a subset of the plurality of user interfaces to present a corresponding portion of the digital therapeutic content to the user;
receiving, by the one or more processors, an indication to provide access to the entirety of the digital therapeutic content to the user; and
providing, by the one or more processors, to the user device, access to the plurality of user interfaces to present an entirety of the digital therapeutic content to the user, responsive to receiving the indication to approve access.

9. The method of claim 8, further comprising:
determining, by the one or more processors, that a time window for providing the user device access to the subset of the plurality of user interfaces has expired; and
restricting, by the one or more processors, the user device from accessing the subset of the plurality of user interfaces, responsive to determining that the time window has expired.

10. The method of claim 8, further comprising generating, by the one or more processors, an access code to provide the user device access to the subset of the plurality of user interfaces, responsive to the indication to provide the digital therapeutic content to the user, and
wherein providing the user device access to the subset of the plurality of user interfaces further comprises providing the user device access to the subset of the plurality of user interfaces, responsive to entry of the access code via the user device.

11. The method of claim 8, further comprising:
selecting, by the one or more processors, from a second plurality of user interfaces, a second user interface to add to the subset of the plurality of user interfaces based on data from the user device, the data identifying interactions by the user with one or more of the subset of the plurality of user interfaces; and
providing, by the one or more processors, to the user device, access to the second user interface in addition to the subset of the plurality of user interfaces.

12. The method of claim 8, further comprising generating, by the one or more processors, an access code to provide the user device access to the plurality of user interfaces for the entirety of the digital therapeutic content, and
wherein providing the user device access to the plurality of user interfaces further comprises providing the user device access to the plurality of user interfaces, responsive to entry of the access code via the user device.

13. The method of claim 8, wherein receiving the indication further comprises receiving the indication responsive to an approval by a remote service of the provision of the entirety of the digital therapeutic content to the user.

14. The method of claim 8, wherein the user is on a medication to address the condition, in at least partial concurrence with at least one of (i) provision of the subset of the plurality of user interfaces for the portion of the digital therapeutic content or (ii) provision of the plurality of user interfaces for the entirety of the digital therapeutic content.

15. The method of claim 8, wherein the condition comprises at least one of: substance use disorder, opioid use disorder, chronic insomnia, alcohol use disorder, schizophrenia, generalized anxiety disorder, major depressive disorder, bipolar, posttraumatic stress disorder, acute and chronic pain, migraine, multiple sclerosis, epilepsy, irritable bowel syndrome, specialty gastroenterology, cancer, or cardiovascular disease.

16. A system for controlling access to user interfaces, the system comprising:
one or more processors coupled with memory, the one or more processors configured to perform the method of any one of claims 1 to 7.

17. A system for controlling access to user interfaces, the system comprising:
one or more processors coupled with memory, the one or more processors configured to perform the method of any one of claims 8 to 15.
